(19) Europäisches Patentamt
European Patent Office
Office européen des brevets

(11) Publication number: **0 478 964 A1**

# (12) EUROPEAN PATENT APPLICATION

(21) Application number: **91114680.1**

(51) Int. Cl.[5]: **C07D 471/04**, A61K 31/435, //(C07D471/04,235:00,221:00)

(22) Date of filing: **30.08.91**

(30) Priority: **06.09.90 JP 237984/90**

(43) Date of publication of application: **08.04.92 Bulletin 92/15**

(84) Designated Contracting States: **AT BE CH DE DK ES FR GB GR IT LI LU NL SE**

(71) Applicant: **SHIONOGI SEIYAKU KABUSHIKI KAISHA**
**1-8, Doshomachi 3-chome Chuo-ku**
**Osaka 541(JP)**

(72) Inventor: **Takada, Susumu**
**4-6-78, Midoridai**
**Kawanishi-shi, Hyogo(JP)**
Inventor: **Chomei, Nobuo**
**1-1216-7, Uenoshibamukougaoka-cho**
**Sakai-shi, Osaka(JP)**
Inventor: **Sasatani, Takashi**
**1-1079-86, Maruyama**
**Nara-shi, Nara(JP)**
Inventor: **Matsushita, Akira**
**2-3-22, Fukaeminami-machi, Higashinada-ku**
**Kobe-shi, Hyogo(JP)**
Inventor: **Eigyo, Masami**
**2-10-7, Shikanodainishi**
**Ikoma-shi, Nara(JP)**

(74) Representative: **Vossius & Partner**
**Siebertstrasse 4 P.O. Box 86 07 67**
**W-8000 München 86(DE)**

## (54) Pyrazoloquinoline derivatives.

(57) Compound of formula:

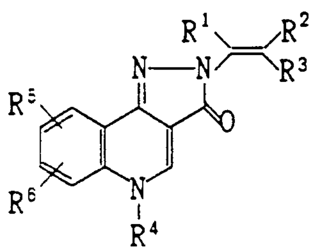

wherein $R^1$, $R^2$, and $R^3$ are hydrogen, alkyl, cycloalkyl, or phenyl or $R^1$ and $R^2$ taken together may form polymethylene, which may be intervened by one or more of oxygen, sulfur, and/or nitrogen and may have 1 or 2 lower alkyls as substituent; $R^4$ is hydrogen or alkyl; $R^5$ and $R^6$ are hydrogen, alkyl, alkyloxy, or halogen, or taken together may form methylenedioxy.

The compounds show agonist, antagonist, or inverse agonist activities depending on the difference of their structure, whereby these are thought to be effective for antianxiety, antagonist against anesthetic, and antiencephalopathy.

EP 0 478 964 A1

The present invention relates to novel substituted pyrazolo-quinoline derivatives which are useful as antianxiety agents, antagonists against anesthetics, and for effecting antiencephalopathy.

Many kinds of compounds which do not belong to the benzodiazepine (BDZ) type have been investigated and disclosed in specifications of JPN Unexamd. Pat. Publn. Nos. 56-18,980 and 61-112,075 as compounds showing high affinity to cerebral BDZ receptors and being effective as psychotropic agents.

The compounds of the present invention show agonist, antagonist, or inverse agonist activities depending on their structure. Therefore, the compounds possessing agonist activities are effective as a sleep-inducer or as antianxiety drugs, the compounds possessing antagonist activities are effective as antagonists against anesthetic agents, and the compounds possessing inverse agonist activities are effective as antagonists against anesthetic agents and as cerebral function stimulating agents.

The present invention relates to compounds of the formula:

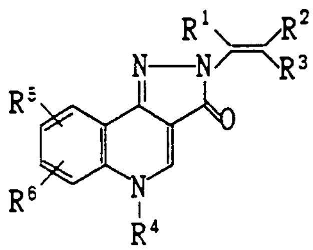

( I )

wherein $R^1$, $R^2$, and $R^3$ each is hydrogen, lower alkyl, cycloalkyl, or phenyl or $R^1$ and $R^2$ taken together may form polymethylene, which may be intervened by one or more of oxygen, sulfur, and/or nitrogen and may have 1 or 2 lower alkyls as substituents; $R^4$ is hydrogen or lower alkyl; $R^5$ and $R^6$ each is hydrogen, lower alkyl, lower alkyloxy, or halogen or taken together may form methylenedioxy,
or salts thereof.

In the specification, the term "lower alkyl" refers to a straight or branched chain $C_1$ to $C_6$ alkyl, including methyl, ethyl, n-propyl, isopropyl, n-butyl, isobutyl, sec-butyl, tert-butyl, 1-methylpropyl, n-pentyl, isopentyl, neopentyl, tert-pentyl, 1-methylbutyl, 2-methylbutyl, 1,1-dimethylpropyl, 2,2-dimethylbutyl, n-hexyl, isohexyl, and the like.

The term "cycloalkyl" refers to $C_3$ to $C_6$ cycloalkyl including cyclopropyl, cyclobutyl, cyclopentyl, and cyclohexyl, most preferably cyclopropyl.

The term "lower alkyloxy" refers to $C_1$ to $C_6$ alkyloxy including methoxy, ethoxy, propoxy, butoxy, pentyloxy, and phenoxy.

The term "polymethylene" refers to $C_2$ to $C_6$ polymethylene including ethylene, trimethylene, tetramethylene, pentamethylene, and hexamethylene to form consequently 4- to 8-membered cycloalkenyl together with the adjacent "vinylene group". Further the polymethylene may be intervened by one or more oxygen, sulfur, and/or nitrogen to form 4- to 8-membered heterocycles, and may have 1 or 2 lower alkyls as substituent, namely, azete, azetine, oxetene, thietene, 2H-pyrrole, oxazole, isoxazole, thiazole, isothiazole, imidazole, pyrazole, pyrroline, pyrazoline, dihydrothiophene, dihydrofuran, pyrazine, pyrimidine, dihydropyran, dihydrothiopyran, tetrahydrothiepin, tetrahydroxepin, tetrahydrothiocin , tetrahydroxocin, preferably dihydrothiophene, dihydrofuran, dihydropyran, and dihydrothiopyran.

The term "halogen" refers to fluorine, chlorine, bromine, and iodine.

The acid addition salts include inorganic acid salts such as the hydrochloride, hydrobromide, and sulfate, and organic acid salts such as the acetate, oxalate, succinate, maleate, fumarate, and benzoate.

The compounds of this invention can be prepared by the following method:

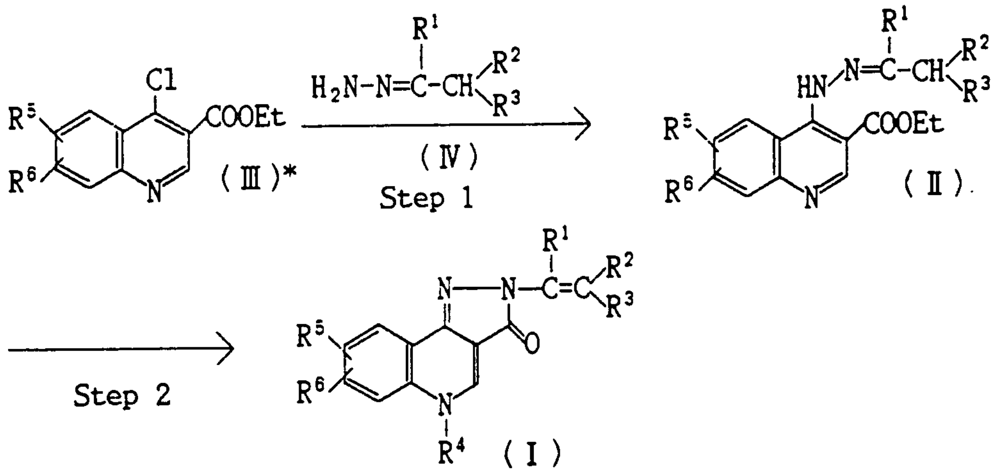


(III)* (IV) (II) (I)

*: J. Am. Chem. Soc., 68, 1264 (1964)
J. Org. Chem., 18, 55 (1953)

In the reaction scheme above, $R^1$,$R^2$,$R^3$,$R^4$,$R^5$,and $R^6$ each has the same meaning as defined above.

## Step 1

The compound (III) is allowed to react with a equimolar or an excessive molar amount of the compound (IV), if necessary under stirring, to give the objective compound (II).

The reaction may be performed at 0-80 °C, preferably from at room temperature to 50 °C for 10 to 40 hours in a solvent, for example, in lower alkanols such as methanol and ethanol, or toluene, xylene, biphenyl, or diphenyl ether.

## Step 2

The compound (II) is allowed to react for the ring closure to give the compound (I).

The reaction may be carried out in an appropriate solvent at 10-100 °C, preferably at room temperature, if necessary under stirring, in the presence of a base such as sodium methoxide, sodium ethoxide, or potassium tert-butoxide.

The solvents which may be used are alcohols such as methanol or ethanol, or halogenocarbons such as dichloromethane, dichloroethane, chloroform, or carbon tetrachloride, aromatic solvents such as benzene, toluene, xylene, or dimethylformamide, dimethyl sulfoxide, most preferably dimethyl sulfoxide.

The compounds of the present invention can be administered orally or parenterally. For example, the compounds of the present invention may be orally administered in a form of tablets, powders, capsules, and granules, solutions or suspensions, or other liquids such as syrups or elixirs, and parenterally in the form of injections of aqueous or oily suspensions.

These preparations can be prepared in a conventional manner by using excipients, binders, lubricants, aqueous or oily solubilizers, emulsifier, suspending agents, and the like. Additionally, preservatives and stabilizers can be used.

The dosages may vary depending upon the administration route, age, weight, condition, and the kind of disease of the patients, but are usually 0.05-500 mg/day, preferably 0.1-200 mg/day through oral route, and 0.01-300 mg/day, preferably 0.05-100 mg/day through parenteral route in a single or divided doses.

The present invention is illustrated by the following examples and reference examples, which are not to be considered as limiting.

The abbreviations used in examples and reference examples have the following meanings.

| | |
|---|---|
| Me : | methyl |
| Et : | ethyl |
| iPr : | isopropyl |
| DMSO : | dimethyl sulfoxide |

Example 1

(1) Ethyl 4-(cyclopentylidenehydrazino)quinoline-3-carboxylate (II-1)

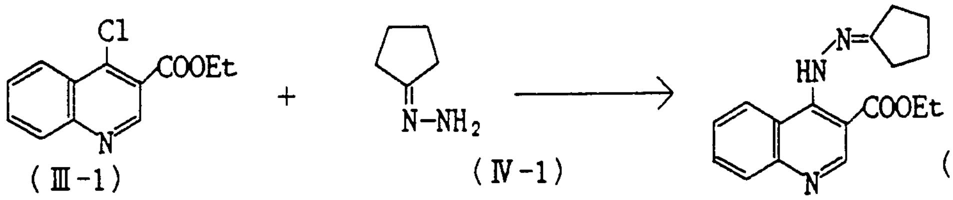

(III-1) (IV-1) (II-1)

To a solution of 708 mg of ethyl 4-chloroquinoline-3-carboxylate (III-1) in 20 ml of ethanol was added 583 mg of cyclopentanone hydrazone under stirring at room temperature and the mixture allowed to stand for 20 hours. The reaction mixture was concentrated under reduced pressure to give a residue, which was dissolved in methylene chloride, alkalified with a saturated aqueous solution of sodium hydrogencarbonate and extracted with methylene chloride. The organic layer was dried and evaporated to give an orange oil, which was purified by silica gel column chromatography (methylene chloride/methanol) to give the compound (II-1) as crude crystals. This was recrystallized from a methanol/ether mixture to give 667mg of the compound (II-1) in 73% yield, yellow crystals, m.p. 132.5-133.5 °C.

| Elementary Analysis (%) for $C_{17}H_{19}N_3O_2$ | | | |
|---|---|---|---|
| Calcd.: | C, 68.66; | H, 6.44; | N, 14.13 |
| Found : | C, 68.51; | H, 6.35; | N, 14.43 |

(2) 2-(Cyclopenten-1-yl)-2,5-dihydro-3H-pyrazolo[4,3-c]quinolin-3-one (I-1)

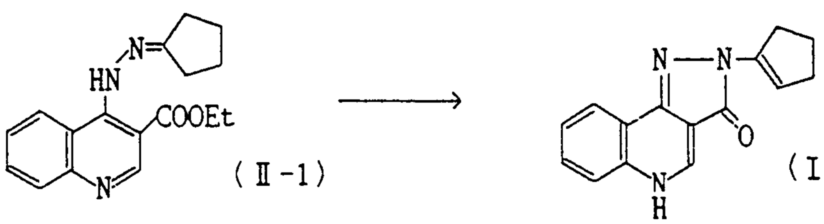

(II-1) (I-1)

To a 10ml solution of 1.02g of the above compound (II-1) in dry dimethyl sulfoxide was added 1.13g of potassium tert-butoxide at room temperature, while being stirred vigorously for 1 hour. To the reddish black reaction mixture was added about 40ml of a saturated aqueous solution of ammonium chloride. The mixture was extracted with several portions of methylene chloride. The organic layers were combined and concentrated to give a residue, which was chromatographed on silica gel (methylene chloride/methanol) to give the compound (I-1) as crude crystals. This was dissolved in methanol under heating and decolorized with active carbon to give 536mg of the compound (I-1) in 71% yield, m.p. 253-255 °C.

| Elementary Analysis (%) for $C_{15}H_{13}N_3O \cdot 1/8H_2O$ | | | |
|---|---|---|---|
| Calcd.: | C, 71.05; | H, 5.27; | N, 16.58 |
| Found : | C, 71.12; | H, 5.19; | N, 16.75 |

Examples 2-7

The objective compounds (I) were obtained, substantially in the same manner as in Example 1 but

under the reaction conditions specified in Table 1. Physical constants of the compounds are shown in Table 2.

Table 1

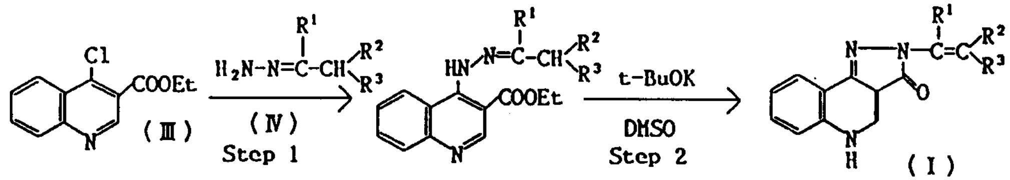


| Ex. No. | Compound (IV) | Step 1 (II) (mg) | (IV) (mg) | EtOH (ml) | rea. time (hr) | Yield (mg) | (%) | Compd. No. | Step 2 t-BuOK (mg) | DMSO (ml) | rea. time (hr) | Yield (mg) | (%) | Compd. No. |
|---|---|---|---|---|---|---|---|---|---|---|---|---|---|---|
| 2 | 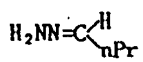 | 710 | 524 | 20 | 24 | 752 | 88 | II-2 | 1014 | 8.6 | 2 | 489 | 68.0 | I-2 |
| 3 | 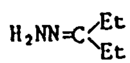 | 709 | 601 | 20 | 18 | 567 | 63 | II-3 | 640 | 5.6 | 2 | 251<br>143 | 33.0<br>19.0 | I-3<br>I-4 |
| 4 | 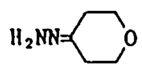 | 1139 | 828 | 20 | 22 | 1119 | 74 | II-4 | 1318 | 12.0 | 1 | 922 | 67.0 | I-5 |
| 5 | 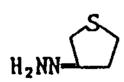 | 1182 | 1184 | 20 | 22.5 | 1081 | 69 | II-5 | 1490 | 12.0 | 1 | 213<br>229 | 18.0<br>19.0 | I-6<br>I-7 |
| 6 | 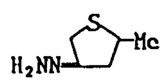 | 1378 | 761 | 20 | 22 | 992 | 52 | II-6 | 811 | 23.7 | 1 | 695<br>496 | 30.0<br>21.0 | I-8<br>I-9 |
| 7 | 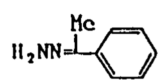 | 709 | 806 | 20 | 24 | 733 | 73 | II-7 | 740 | 7.30 | 2 | 455 | 52.0 | I-10 |

Table 2 (No. 1)

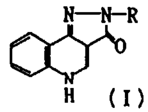

(I)

| Compd. No. | Compound (II) m.p (°C) | Compound (II) recrystallization solvent | Compound (II) Elemental Analysis (%) Up (Calcd.) Down (Found) | Compound (I) Comp No. | Compound (I) substituent R | Compound (I) m.p. (°C) | Compound (I) recrystallization solvent | Compound (I) Elementary Analysis (%) Up (Calcd.) Down (Found) |
|---|---|---|---|---|---|---|---|---|
| II-2 | 89-92 | n-hexane | $C_{16}H_{19}N_3O_2$ C, 67.34: H, 6.71 N, 14.73 / C, 67.62: H, 6.79 N, 14.90 | I-2 | 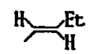 (E) | 202-204 | methanol-dichloroethane | $C_{14}H_{13}N_3O \cdot 1/4H_2O$ C, 68.98: H, 5.58 N, 17.24 / C, 69.09: H, 5.73 N, 17.39 |
| II-3 | 120-121 | methanol-ether | $C_{17}H_{21}N_3O_2$ C, 68.20: H, 7.07 N, 14.04 / C, 68.11: H, 7.05 N, 14.24 | I-3 | 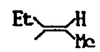 (Z) | 219-222 | n-hexane-ether | $C_{15}H_{15}N_3O \cdot 1/4H_2O$ C, 69.88: H, 6.06 N, 16.30 / C, 69.89: H, 6.21 N, 16.34 |
| | | | | I-4 | 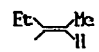 (E) | 239.5-241 | n-hexane-ether | $C_{15}H_{15}N_3O \cdot 1/4H_2O$ C, 69.88: H, 6.06 N, 16.30 / C, 69.59: H, 6.21 N, 16.34 |
| II-4 | 174-175 | methanol-dichloroethane | $C_{17}H_{19}N_3O_3$ C, 65.16: H, 6.11 N, 13.41 / C, 65.09: H, 6.17 N, 13.49 | I-5 | 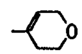 | 265-267.5 (dec.) | methanol-dichloroethane | $C_{15}H_{13}N_3O_2$ C, 67.40: H, 4.90 N, 15.72 / C, 67.28: H, 4.97 N, 15.82 |
| II-5 | 120-122 | methanol | $C_{16}H_{17}N_3O_2S$ C, 60.93: H, 5.43 N, 13.32: S, 10.16 / C, 60.64: H, 5.52 N, 13.59: S, 10.17 | I-6 | 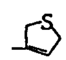 | 289-294 (dec.) | methanol-dichloromethane | $C_{14}H_{11}N_3OS \cdot H_2O$ C, 58.52: H, 4.56 N, 14.62: S, 11.16 / C, 58.40: H, 4.70 N, 14.47: S, 11.26 |
| | | | | I-7 | 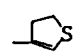 | 295-298 (dec.) | methanol-dichloromethane | $C_{14}H_{11}N_3OS \cdot H_2O$ C, 58.52: H, 4.56 N, 14.62: S, 11.16 / C, 58.77: H, 4.71 N, 14.51: S, 10.86 |

Table 2 (No. 2)

| Compd. No. | Compound (II) m.p. (°C) | Compound (II) recrystallization solvent | Compound (II) Elementary Analysis (%) Up (Calcd.) Down (Found) | Compound (I) Compd. No. | Compound (I) Substituent R | Compound (I) m.p. (°C) | Compound (I) recrystallization solvent | Compound (I) Elementary Analysis (%) Up (Calcd.) Down (Found) |
|---|---|---|---|---|---|---|---|---|
| II-6 | 123 -125 | methanol | $C_{17}H_{19}N_3O_2S$ C, 61.98; H, 5.81 N, 12.75; S, 9.73<br>C, 61.70; H, 5.73 N, 12.80; S, 9.71 | I-8 | 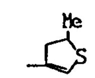 | 254 -256 | methanol | $C_{15}H_{13}N_3OS \cdot 1/2CH_3OH \cdot 1/5H_2O$ C, 61.45; H, 5.12 N, 13.87; S, 10.58<br>C, 61.51; H, 5.06 N, 13.67; S, 10.64 |
| | | | | I-9 | 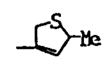 | 229 -233 | methanol-dichloroethane | $C_{15}H_{13}N_3OS \cdot 2/5H_2O$ C, 62.01; H, 4.79 N, 14.46; S, 11.04<br>C, 61.95; H, 4.87 N, 14.26; S, 10.96 |
| II-7 | 169 -170 | n-hexane-methanol | $C_{18}H_{13}N_3O \cdot 1/4H_2O$ C, 74.09; H, 4.66 N, 14.40<br>C, 74.24; H, 4.66 N, 14.11 | I-10 | 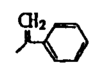 | 213 -218 | methanol | $C_{18}H_{13}N_3O \cdot 1/4H_2O$ C, 74.09; H, 4.66 N, 14.40<br>C, 74.24; H, 4.66 N, 14.11 |

Example 8

(1) Ethyl 6-methoxy-4-(tetrahydrofuranylidenehydradino)quinolin-3-carboxylate (II-8)

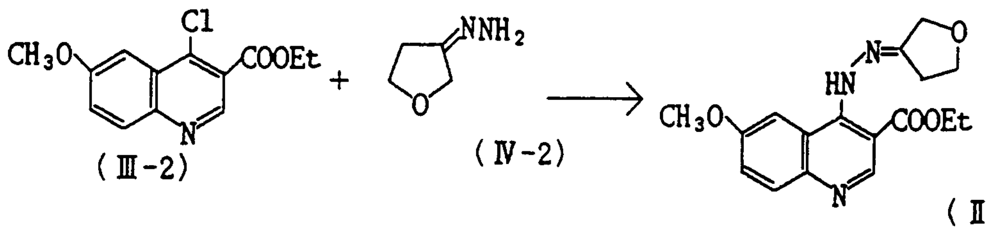

(III-2) (IV-2) (II-8)

In a 30ml solution of 3.91g of tetrahydrofuran-3-one hydrazone (IV-2) in dry ethanol was added 6.91g of ethyl 6-methoxy-4-chloroquinolinecarboxylate (III-2), while being stirred at room temperature. The reaction mixture was allowed to stand overnight and refluxed for 2 hours under heating and then concentrated under reduced pressure to give a residue. The red residue was alkalified with a saturated aqueous solution of sodium hydrogen carbonate and extracted with methylene chloride. The organic layer was evaporated under reduced pressure to give a residue, which was chromatographed on silica gel (methylene chloride/methanol) to give 6.93g of the compound (II-8) as crude crystals.

(2) 8-Methoxy-2-(2,5-dihydrofuran-3-yl)-2,5-dihydro-3H-pyrazolo[4,3-c]quinolin-3-one (I-11)

(II-8)

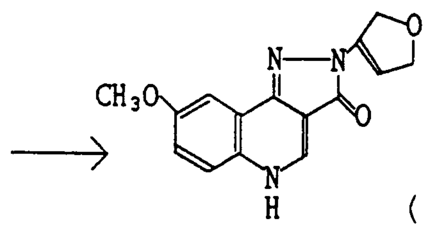

(I-11)

To a solution of a compound (II-8) obtained by the first process in dry dimethyl sulfoxide 70ml was added 7.90g of potassium tert-butoxide under stirring at room temperature and the mixture allowed to stand for 2 hours. To the reaction mixture was added 150ml of a saturated aqueous solution of ammonium chloride and allowed to stand for 30 minutes to give yellow precipitates, which were collected by filtration, the filtrate was extracted with methylene chloride. The extracts and the precipitates were combined and chromatographed on silica gel (methylene chloride/methanol) to give the compound (I-11) as crude crystals. This was dissolved in a methylene chloride-methanol mixed solution under heating and decolorized with active carbon and recrystallized to give 4.85g of the compound (I-11) in 73% yield, yellow needles, m.p. 263.5-266 °C.

| Elementary Analysis (%) for $C_{15}H_{13}N_3O_3$ | | | |
|---|---|---|---|
| Calcd.: | C, 62.02; | H, 4.79; | N, 14.46 |
| Found : | C, 62.02; | H, 4.77; | N, 14.30 |

Example 9-42

The objective compounds (I) were obtained, substantially in the same manner as in Example 8 but under the reaction conditions as specified in Table 3. Physical constants of the compounds are shown in Table 4.

Table 3 (No. 1)

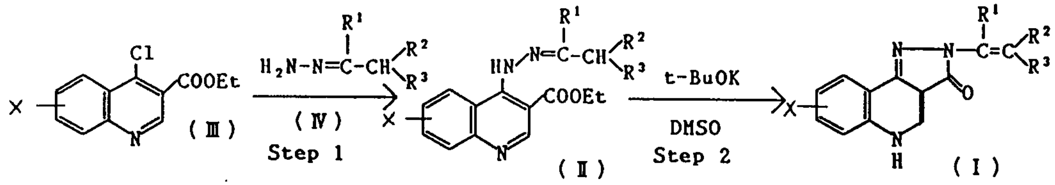


| Ex. No. | Compound (IV') | Step 1 (III) (mg) | (IV) (mg) | EtOH (ml) | rea. time (hr) | Compd. No. | Step 2 t-BuOK (mg) | DMSO (ml) | rea. time (hr) | Yield (mg) | (%) | Compd. No. |
|---|---|---|---|---|---|---|---|---|---|---|---|---|
| 9 | 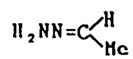 | 1887 | 2326 | 10 | 28.5 | II -9 | 1351 | 10.4 | 1 | 660 | 41 | I -12 |
| 10 | 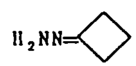 | 709 | 519 | 20 | 22 | II -10 | 1822 | 8.1 | 16 (50°C) | 497 | 77 | I -13 |
| 11 | 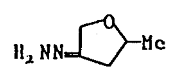 | 2145 | 2082 | 30 | 18 | II -11 | 1951 | 18.0 | 1 | 548 | 35 | I -14 |
| 12 | 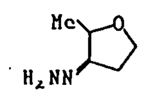 | 3206 | 3116 | 30 | 14 | II -12 | 3411 | 31.0 | 1 | 1094 | 40 | I -15 |
| 13 | 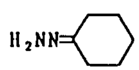 | 2357 | 1683 | 20 | 18 | II -13 | 3367 | 16.0 | 2 | 1968 | 74 | I -16 |
| 14 | 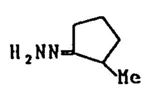 | 955 | 906 | 15 | 22.5 | II -14 | 1347 | 14.0 | 1 | 233<br>569 | 22<br>53 | I -17<br>I -18 |

Table 3 (No. 2)

| Ex. No. | Compound ( IV ) | Step 1 (III) (mg) | (IV) (mg) | EtOH (ml) | rea.time (hr) | Compd. No. | Step 2 t-BuOK (mg) | DMSO (ml) | rea.time (hr) | Yield (mg) | (%) | Compd. No. |
|---|---|---|---|---|---|---|---|---|---|---|---|---|
| 15 | 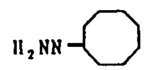 | 712 | 841 | 20 | 27 | II -15 | 1176 | 11.8 | 1 | 473 | 54 | I -19 |
| 16 | 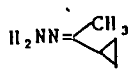 | 720 | 602 | 20 | 24 | II -16 | 1037 | 9.2 | 1 | 48 | 6 | I -20 |
| 17 | 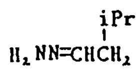 | 712 | 601 | 20 | 22 | II -17 | 1010 | 10.0 | 2 | 167<br>124 | 22<br>16 | I -21<br>I -22 |
| 18 | 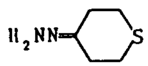 | 714 | 844 | 20 | 24 | II -18 | 1052 | 10.3 | 1 | 279 | 33 | I -23 |
| 19 | 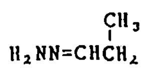 | 1119 | 682 | 30 | 26 | II -19 | 1595 | 12.8 | 2 | 407 | 38 | I -24 |
| 20 | 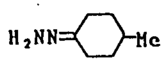 | 714 | 1421 | 20 | 22 | II -20 | 1023 | 8.0 | 1 | 131 | 16 | I -25 |
| 21 | 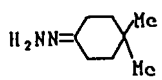 | 1178 | 1409 | 15 | 23 | II -21 | 1827 | 10.0 | 1 | 495 | 34 | I -26 |

Table 3 (No. 3)

| Ex. No. | Compound (Ⅳ) | Step 1 (Ⅲ) (mg) | Step 1 (Ⅳ) (mg) | Step 1 EtOH (ml) | Step 1 rea. time (hr) | Step 1 Compd. No. | Step 2 t-BuOK (mg) | Step 2 DMSO (ml) | Step 2 rea. time (hr) | Step 2 Yield (mg) | Step 2 Yield (%) | Step 2 Compd. No. |
|---|---|---|---|---|---|---|---|---|---|---|---|---|
| 22 | 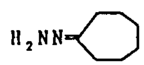 | 760 | 707 | 15 | 19.5 | Ⅱ-22 | 1010 | 12.2 | 1 | 423 | 45 | Ⅰ-27 |
| 23 | 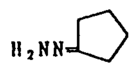 | 810 | 592 | 15 | 23 | Ⅱ-23 | 1015 | 10.0 | 1 | 563 | 65 | Ⅰ-28 |
| 24 | 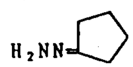 | 812 | 592 | 15 | 22 | Ⅱ-24 | 1013 | 10.0 | 1 | 625 | 72 | Ⅰ-29 |
| 25 | 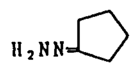 | 810 | 590 | 15 | 22 | Ⅱ-25 | 1033 | 12.0 | 1 | 636 | 70 | Ⅰ-30 |
| 26 | 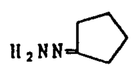 | 721 | 596 | 15 | 21.5 | Ⅱ-26 | 1016 | 10.0 | 1 | 636 | 57 | Ⅰ-31 |
| 27 | 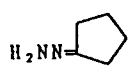 | 720 | 593 | 15 | 22 | Ⅱ-27 | 1019 | 10.0 | 1 | 495 | 61 | Ⅰ-32 |
| 28 | 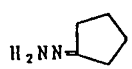 | 679 | 534 | 15 | 24 | Ⅱ-28 | 1010 | 10.0 | 1 | 465 | 55 | Ⅰ-33 |
| 29 | 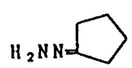 | 247 | 216 | 8 | 24 | Ⅱ-29 | 632 | 7.5 | 1 | 200 | 72 | Ⅰ-34 |

Table 3 (No.4)

| Ex.No. | Compound (Ⅳ) | Step 1 (Ⅲ) (mg) | (Ⅳ) (mg) | EtOH (ml) | rea.time (hr) | Compd. No. | Step 2 t-BuOK (mg) | DMSO (ml) | rea.time (hr) | Yield (mg) | (%) | Compd. No. |
|---|---|---|---|---|---|---|---|---|---|---|---|---|
| 30 | 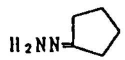 | 802 | 593 | 15 | 22 | Ⅱ-30 | 1105 | 10.0 | 1 | 490 | 55 | Ⅰ-35 |
| 31 | 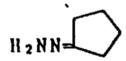 | 708 | 592 | 15 | 24 | Ⅱ-31 | 1011 | 10.0 | 1 | 435 | 54 | Ⅰ-36 |
| 32 | 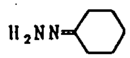 | 798 | 679 | 15 | 18 | Ⅱ-32 | 1012 | 10.0 | 1 | 168 | 19 | Ⅰ-37 |
| 33 | 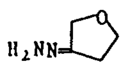 | 1906 | 900 | 20 | 17 | Ⅱ-33 | 2594 | 12.0 | 2 | 1352 | 66 | Ⅰ-38 |
| 34 | 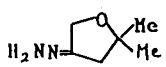 | 2400 | 1742 | 25 | 20 | Ⅱ-34 | 3431 | 20.0 | 1.1 | 1675 | 58 | Ⅰ-39 |
| 35 | 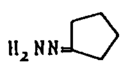 | 1124 | 589 | 20 | 19 | Ⅱ-35 | 1348 | 10.0 | 1.5 | 841 | 72 | Ⅰ-40 |
| 36 | 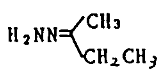 | 943 | 947 | 15 | 24 | Ⅱ-36 | 1324 | 10.0 | 1.5 | 358 | 37 | Ⅰ-41 |

Table 3 (No. 5)

| Ex. No. | Compound (Ⅳ) | Step 1 (Ⅲ) (mg) | Step 1 (Ⅳ) (mg) | Step 1 EtOH (ml) | Step 1 rea. time (hr) | Step 1 Compd. No. | Step 2 t-BuOK (mg) | Step 2 DMSO (ml) | Step 2 rea. time (hr) | Step 2 Yield (mg) | Step 2 Yield (%) | Step 2 Compd. No. |
|---|---|---|---|---|---|---|---|---|---|---|---|---|
| 37 | 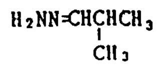 | 943 | 946 | 15 | 24 | Ⅱ-37 | 1506 | 10.0 | 1.67 | 564 | 59 | Ⅰ-42 |
| 38 | 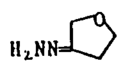 | 1113 | 652 | 20 | 70.3 | Ⅱ-38 | 1352 | 10.0 | 1.3 | 683 | 58 | Ⅰ-43 |
| 39 | 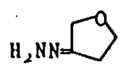 | 750 | 472 | 20 | 67.5 | Ⅱ-39 | 1009 | 10.0 | 1 | 474 | 55 | Ⅰ-44 |
| 40 | 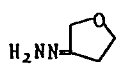 | 1108 | 624 | 25 | 71 | Ⅱ-40 | 1429 | 13.0 | 1.5 | 615 | 52 | Ⅰ-45 |
| 41 | 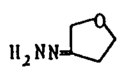 | 662 | 394 | 20 | 69 | Ⅱ-41 | 882 | 9.8 | 1.5 | 417 | 59 | Ⅰ-46 |
| 42 | 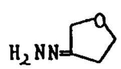 | 458 | 266 | 15.5 | 72 | Ⅱ-42 | 570 | 6.0 | 1.25 | 286 | 59 | Ⅰ-47 |

Table 4 (No.1)

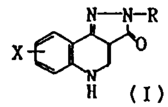

(I)

| Compd. No. | substituent | | m.p. | appearance | Solvent for recrystallization | Elementary Analysis (%) Up (Calcd.) Down (Found) | |
|---|---|---|---|---|---|---|---|
| | X | R | | | | | |
| I − 12 | H | -CH=$CH_2$ | 273 -275 (dec.) | yellow | methanol-dichloro-methane | $C_{12}H_9N_3O$ | C,68.23; H,4.30 N,19.90<br>C,68.13; H,4.49 N,19.97 |
| I − 13 | H | 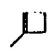 | 263 -265 (dec.) | yellow | methanol-dichloro-methane | $C_{14}H_{11}N_3O \cdot 2/5H_2O$ | C,68.78; H,4.86 N,17.19<br>C,68.80; H,5.15 N,16.79 |
| I − 14 | H | 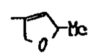 | 256 -259 | yellow | methanol-dichloro-methane | $C_{15}H_{13}N_3O_2 \cdot 1/8H_2O$ | C,66.84; H,4.95 N,15.59<br>C,67.03; H,5.12 N,15.72 |
| I − 15 | H | 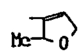 | 230 -232.5 | yellow | methanol-dichloro-methane | $C_{15}H_{13}N_3O_2$ | C,67.40; H,4.90 N,15.72<br>C,67.18; H,5.13 N,15.86 |
| I − 16 | H | 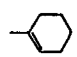 | 275.5 -277 | yellow | methanol | $C_{16}H_{15}N_3O$ | C,72.43; H,5.70 N,15.80<br>C,72.51; H,5.90 N,15.99 |
| I − 17 | H | 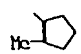 | 275 -278 | light yellow | methanol-ether | $C_{16}H_{15}N_3O \cdot 1/4H_2O$ | C,71.23; H,5.79 N,15.57<br>C,71.28; H,6.06 N,15.56 |

Table 4 (No. 2)

| Compd. No. | Substituent X | Substituent R | m.p. (°C) | appearance | Solvent for recrystallization | Elemental Analysis (%) Up (Calcd.) Down (Found) | |
|---|---|---|---|---|---|---|---|
| I − 18 | H | 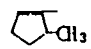 | 257 -259 | orange | methanol-dichloromethane | $C_{16}H_{15}N_3O$ | C.72.43: H.5.69 N.15.83<br>C.72.25: H.5.85 N.15.85 |
| I − 19 | H | 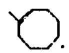 | 281 -283 | orange | methanol | $C_{18}H_{19}N_3O$ | C.73.69: H.6.53 N.14.33<br>C.73.60: H.6.60 N.14.46 |
| I − 20 | H | 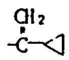 | 202 -204 | orange | methanol | $C_{15}H_{13}N_3O \cdot 1/8H_2O$ | C.71.05: H.5.27 N.16.58<br>C.71.16: H.5.18 N.16.90 |
| I − 21 | H | 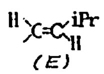 (E) | 215.5-217.5 | yellow | methanol | $C_{15}H_{15}N_3O \cdot 1/5H_2O$ | C.70.13: H.6.04 N.16.36<br>C.69.93: H.6.07 N.16.51 |
| I − 22 | H | 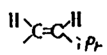 (Z) | 215 -217 | yellow | methanol | $C_{15}H_{15}N_3O \cdot 1/4H_2O$ | C.69.88: H.6.06 N.16.30<br>C.69.73: H.6.12 N.16.37 |
| I − 23 | H | 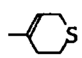 | 262.5 -264 (dec.) | orange | methanol | $C_{15}H_{13}N_3OS \cdot 2/5H_2O$ | C.62.01: H.4.79 N.14.46: S.11.04<br>C.61.99: H.4.87 N.14.47: S.10.95 |
| I − 24 | H | 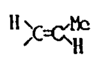 (E) | 228 -230 | yellow | methanol-dichloromethane | $C_{13}H_{11}N_3O \cdot 1/3H_2O$ | C.67.51: H.5.09 N.18.17<br>C.67.65: H.5.16 N.18.23 |

Table 4 (No. 3)

| Compd. No. | Substituent X | Substituent R | m.p. (°C) | appearance | solvent for recrystallization | Elementary Analysis (%) Up (Calcd.) Down (Found) | |
|---|---|---|---|---|---|---|---|
| I − 25 | H | 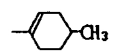 | 265.5-267.5 | orange | methanol-dichloromethane | $C_{17}H_{17}N_3O$ · $1/5H_2O$ | C.72.17: H.6.20 N.14.85<br>C.72.16: H.6.31 N.15.02 |
| I − 26 | H | 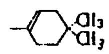 | 291-293 | yellow | methanol-dichloromethane | $C_{18}H_{19}N_3O$ · $1/3H_2O$ | C.72.22: H.6.62 N.14.04<br>C.72.24: H.6.62 N.14.12 |
| I − 27 | H | 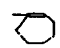 | 242-245 | yellow | methanol | $C_{17}H_{17}N_3O$ · $2/3H_2O$ | C.70.08: H.6.34 N.14.42<br>C.70.20: H.6.43 N.14.71 |
| I − 28 | 8-Cl | 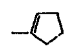 | 266-272 | orange | methanol-dichloromethane | $C_{15}H_{12}N_3OCl$ · $1/8H_2O$ | C.62.56: H.4.29 N.14.59: Cl.12.31<br>C.62.41: H.4.48 N.14.52: Cl.11.59 |
| I − 29 | 7-Cl | 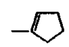 | 275-277 | orange | methanol-dichloromethane | $C_{15}H_{12}N_3OCl$ · $1/8H_2O$ | C.62.56: H.4.29 N.14.59: Cl.12.31<br>C.62.65: H.4.39 N.14.85: Cl.12.38 |
| I − 30 | 6-Cl | 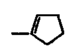 | 260.5-263.0 | orange | methanol-dichloromethane | $C_{15}H_{12}N_3OCl$ · $H_2O$ | C.59.31: H.4.65 N.13.83: Cl.11.67<br>C.59.46: H.4.69 N.13.89: Cl.11.59 |
| I − 31 | 8-F | 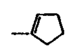 | 260.5-263.0 | yellow | methanol-dichloromethane | $C_{15}H_{12}N_3OF$ · $1/8H_2O$ | C.66.35: H.4.55 N.15.48: F.7.00<br>C.66.23: H.4.70 N.15.41: F.7.11 |
| I − 32 | 7-F | 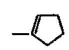 | 256-258.5 | yellow | methanol-dichloromethane | $C_{15}H_{12}N_3OF$ | C.66.90: H.4.49 N.15.60: F.7.05<br>C.66.62: H.4.64 N.15.52: F.6.92 |

Table 4 (No. 4)

| Compd. No. | Substituent | | m.p. (°C) | appearance | Solvent for recrystallization | Elementary Analysis (%) Up (Calcd.) Down (Found) | |
|---|---|---|---|---|---|---|---|
| | X | R | | | | | |
| I - 33 | 8-$OCH_3$ | 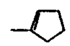 | 266 -268 | orange | methanol-dichloro-methane | $C_{16}H_{15}N_3O_2$ | C, 68.31: H, 5.37 N, 14.93<br>C, 68.10: H, 5.52 N, 14.95 |
| I - 34 | 7-$OCH_3$ | 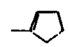 | 265.0-267.5 (dec.) | orange | methanol-dichloro-methane | $C_{16}H_{15}N_3O_2 \cdot 1/6H_2O$ | C, 67.59: H, 5.44 N, 14.78<br>C, 67.50: H, 5.34 N, 14.80 |
| I - 35 | 7,8-$-OCH_2O$ | 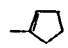 | >300 | orange | methanol-dichloro-methane | $C_{16}H_{13}N_3O_3$ | C, 65.07: H, 4.43 N, 14.22<br>C, 64.79: H, 4.50 N, 14.23 |
| I - 36 | 8-$CH_3$ | 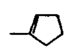 | 282 -285 (dec.) | yellow | methanol-dichloro-methane | $C_{16}H_{15}N_3O \cdot 1/8H_2O$ | C, 71.82: H, 5.74 N, 15.70<br>C, 72.07: H, 5.73 N, 15.77 |
| I - 37 | 7-$OCH_3$ | 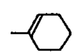 | 256 -259 | red-dish orange | methanol-dichloro-methane | $C_{17}H_{17}N_3O_2 \cdot 1/8H_2O$ | C, 68.61: H, 5.84 N, 14.12<br>C, 68.48: H, 5.88 N, 14.31 |
| I - 38 | H | 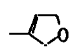 | 276 -278 (dec.) | orange | methanol-dichloro-methane | $C_{14}H_{11}N_3O_2$ | C, 66.39: H, 4.37 N, 16.59<br>C, 66.25: H, 4.55 N, 16.71 |
| I - 39 | H | 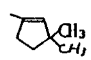 | 251-253.5 (dec.) | yellow | methanol-dichloro-methane | $C_{16}H_{15}N_3O_2 \cdot H_2O$ | C, 64.20: H, 5.72 N, 14.04<br>C, 64.22: H, 5.30 N, 13.93 |

Table 4 (No.5)

| Compd. No. | Substituent X | Substituent R | m.p. (°C) | appearance | Solvent for recrystallization | Elementary Analysis (%) Up (Calcd.) Down (Found) | |
|---|---|---|---|---|---|---|---|
| I − 40 | 8-iPr | 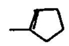 | 244 -246 | orange | methanol-dichloromethane | $C_{18}H_{19}N_3O \cdot 1/4H_2O$ | C,72.58; H,6.60 N,14.11<br>C,72.55; H,6.76 N,14.22 |
| I − 41 | H | 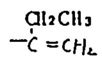 | 164 -167 | orange | methanol-dichloromethane | $C_{14}H_{13}N_3O \cdot 1/8H_2O$ | C,69.62; H,5.53 N,17.40<br>C,69.33; H,5.49 N,17.57 |
| I − 42 | H | 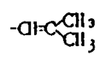 | >300 | orange | methanol-dichloromethane | $C_{14}H_{13}N_3O$ | C,70.27; H,5.48 N,17.57<br>C,70.26; H,5.50 N,17.75 |
| I − 43 | 8-iPr | 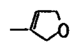 | 258 -260 | yellow | methanol | $C_{17}H_{17}N_3O_2 \cdot 1/3H_2O$ | C,67.75; H,5.91 N,13.95<br>C,67.99; H,6.01 N,14.10 |
| I − 44 | 8-CH₃ | 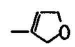 | 266 -269 (dec.) | yellow | methanol | $C_{15}H_{13}N_3O_2 \cdot H_2O$ | C,63.15; H,5.30 N,14.73<br>C,63.34; H,5.39 N,14.96 |
| I − 45 | 7-OCH₃ | 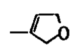 | 267 -269 (dec.) | yellow | methanol-dichloromethane | $C_{15}H_{13}N_3O_3 \cdot 1/8H_2O$ | C,63.10; H,4.68 N,14.72<br>C,63.00; H,4.77 N,14.98 |
| I − 46 | 8-F | 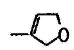 | 276.5 -278.0 | yellow | methanol-dichloromethane | $C_{14}H_{10}N_3O_2F \cdot 1/8H_2O$ | C,61.48; H,3.78 N,15.36; F,6.95<br>C,61.34; H,4.01 N,15.51; F,7.05 |
| I − 47 | 8-Cl | 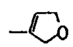 | >300 | yellow | methanol | $C_{14}H_{10}N_3O_2Cl$ | C,58.44; H,3.50 N,14.60; Cl,12.32<br>C,58.24; H,3.69 N,14.83; Cl,12.35 |

## Example 43

### Alternative Process for the compound (I-1)

The compound (I-1) of Example 1 was alternatively obtained by the following process. To an ethanolic solution of sodium ethylate (prepared from 10ml of ethanol and 71mg of metallic sodium) were added

298mg of ethyl 4-(cyclopentylidenehydradino)quinoline-3-carboxylate (II-1) at room temperature and the mixture allowed to stand for 20 hours. The reaction mixture was concentrated under reduced pressure to give a residue, to which a saturated aqueous solution of ammonium chloride was added, and extracted with methylene chloride. The organic layer was dried and concentrated to give a residue, which was recrystallized from a methanol/methylene chloride mixture to give 213mg of the compound (I-1) as crystals in 85% yield. This was identified by the spectrum data to be the compound (I-1) obtained in Example 1.

Reference Example 1

Cyclopentanone hydrazone (IV-1)

To a 15ml solution of 1.27g of cyclopentanone in methylene chloride were added 7ml of hydrazone hydrate at room temperature and the mixture allowed to stand for 17 hours. The reaction mixture was added with saturated brine and extracted with methylene chloride. The organic layer was washed with saturated brine, dried over magnesium sulfate and evaporated to give 1.19g of the objective compound (IV-1) as a colorless oil. This was, without purification, employed for the reaction (1st Step) of Example 1.

Effect of the Invention

The compounds of the present invention show high affinity to cerebral BDZ receptors. The resuit of various investigations is that the compounds which bond to these receptors are classified into the following groups, depending upon the activity.

(1) full agonist:
suppression of the central nervous system, antianxiety, and anti-convulsant activity.
(2) partial agonist:
selective antianxiety
(3) antagonist:
antagonist activity which shows both sides
(4) partial inverse agonist:
stimulation of the central nervous system, stimulation of convulsant activity, antagonist activity against anesthetics
(5) inverse agonist:
the inducement of convulsant activity and anxiety

Further, it is known that these compounds are classified into any one of the groups above by the strength of the suppression or increase of convulsion depending on the dose of pentylenetetrazol [C. Braestrup et al.,Biochem. Pharmcol. 33, 859 (1984)]. It is suggested that the partial inverse agonists can be effective drugs on the memory effects. The major reason is that an inverse agonist, methyl β-carboline-3-carboxylate (β-CCM), facilitates the memory and the learning process of mammals, while diazepam, known as an agonist, seems to be harmful to the memory process of humans[M.Sarter et al.,TINS 11, 13 (19 88)]. Among the compounds of the invention, therefore, those having agonists activities are expected to exhibit antianxiety or anti-convulsant activity, those having antagonistic activities to be agents for treating benzodiazepine accidental supernumeracy intake, and those having inverse agonistic activities to exhibit antiencephalopathy, antiamnesia, and antagonist activity against anesthetics.

Experiments for assessing biological activities of the compounds of the present invention are shown below; the test compound number correspond to those used in Examples and Tables.

Experiment 1

BDZ Receptor Binding Assay

This test was carried out according to the modified method of Möhler & Okada, Science, 198, 849-851 (1977).

The BDZ receptor preparation was provided from the cerebral cortex of Wistar rats (male, 11 to 13 week-old). Inhibitory activities of the test compound were determined by the specific binding strengh of tritium labeled diazepam to the receptor. 2nM tritium labeled diazepam and an aqeuous solution of the test compound at 5 or 6 different concentrations were incubated at 0 °C for 60 minutes. The 50 % inhibitory concentration ($IC_{50}$) was measured by the concentration-response curve.

The inhibitory constant (Ki) was calculated according to the following equation, in which Kd is the

dissociation constant of the tritium labeled diazepam and L is the concentration of the labeled ligand.

$$Ki = \frac{IC_{50}}{1 + L/Kd}$$

The results are shown in the Table 5.

Table 5

| Compd. No. | Ki (nM) |
|---|---|
| I-1 | 0.52 |
| I-6 | 0.67 |
| I-11 | 0.69 |
| I-16 | 1.50 |
| I-21 | 0.98 |
| I-24 | 1.00 |
| I-25 | 3.10 |
| I-31 | 0.48 |
| I-34 | 2.10 |
| I-36 | 0.48 |
| I-38 | 0.59 |
| I-39 | 0.29 |

Experiment 2

The test of suppressing convulsion induced by pentylenetetrazol

Agonist activity was evaluated by the following procedure. A certain amount of a test compound was administered intravenously to 8-16 male mice per group, followed by pentylenetetrazol at a subcuataneous dose of 125 mg/kg. The dose ($ED_{50}$) at which 50% of mice was alive, was calculated by the Probit method. The results are shown in the table 6.

Table 6

| Test Compd. | $ED_{50}$ (mg/kg) |
|---|---|
| I-21 | 0.72 |
| I-25 | 0.69 |
| I-39 | 0.19 |

Experiment 3

The test of potentiating convulsion induced by pentylenetetrazol

Inverse agonist activity was evaluated by the following procedure.
A certain amount of a test compound was administered intravenously to 8-16 male mice per group, followed by pentylenetetrazol at a subcuataneous dose of 90 mg/kg. The dose ($ED_{50}$) at which 50% of mice stayed alive, was calculated by the Probit method. The results are shown in the table 7.

Table 7

| Test Compd. | $ED_{50}$ (mg/kg) |
|---|---|
| I-1 | 1.07 |
| I-6 | 0.52 |
| I-24 | 0.23 |
| I-31 | 0.81 |
| I-36 | 0.40 |

From the above results it is evident that the compounds of the present invention show a high affinity to the benzodiazepine receptors, and act suppressively or stimulatory.

## Claims

**1.** A compound of the formula (I):

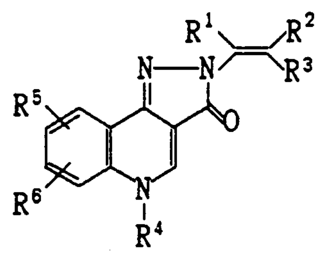

( I )

wherein $R^1$, $R^2$, and $R^3$ each is hydrogen, lower alkyl, cycloalkyl, or phenyl or $R^1$ and $R^2$ taken together may form polymethylene, which may be intervened by one or more of oxygen, sulfur, and/or nitrogen and may have 1 or 2 lower alkyls as substituent; $R^4$ is hydrogen or lower alkyl;
$R^5$ and $R^6$ each is hydrogen, lower alkyl, lower alkyloxy, or halogen, or taken together may form methylenedioxy,
or salts thereof.

**2.** The compound claimed in claim 1, wherein $R^1$ and $R^2$ taken together form polymethylene.

**3.** The compound claimed in claim 2, wherein polymethylene is intervened by one or more oxygen, sulfur, and/or nitrogen.

**4.** The compound claimed in claim 3, wherein polymethylene is intervened by one or more oxygen.

**5.** The compound claimed in claim 3, wherein polymethylene is intervened by one or more sulfur.

**6.** The compound claimed in claim 3, wherein polymethylene is intervened by one or more nitrogen.

**7.** The compound claimed in claim 3, wherein polymethylene may have 1 or 2 lower alkyls as substituents.

**8.** A pharmaceutical composition comprising a pharmacologically effective amount of the compound claimed in any of claims 1 to 7 together with a carrier, diluent, and/or excipient.

**9.** A pharmaceutical composition claimed in claim 8, which is effective as a psychotropic drug.

**10.** Use of a compound of any of claims 1 to 7 for the preparation of a psychotropic drug.

### Claims for the following Contracting State: GR

**1.** A compound of the formula (I):

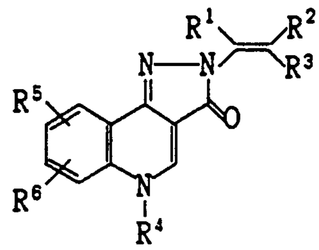

( I )

wherein $R^1$, $R^2$, and $R^3$ each is hydrogen, lower alkyl, cycloalkyl, or phenyl or $R^1$ and $R^2$ taken together may form polymethylene, which may be intervened by one or more of oxygen, sulfur, and/or nitrogen and may have 1 or 2 lower alkyls as substituent; $R^4$ is hydrogen or lower alkyl;
$R^5$ and $R^6$ each is hydrogen, lower alkyl, lower alkyloxy, or halogen, or taken together may form methylenedioxy,
or salts thereof.

**2.** The compound claimed in claim 1, wherein $R^1$ and $R^2$ taken together form polymethylene.

**3.** The compound claimed in claim 2, wherein polymethylene is intervened by one or more oxygen, sulfur, and/or nitrogen.

**4.** The compound claimed in claim 3, wherein polymethylene is intervened by one or more oxygen.

**5.** The compound claimed in claim 3, wherein polymethylene is intervened by one or more sulfur.

**6.** The compound claimed in claim 3, wherein polymethylene is intervened by one or more nitrogen.

**7.** The compound claimed in claim 3, wherein polymethylene may have 1 or 2 lower alkyls as substituents.

**8.** Use of a compound of any of claims 1 to 7 for the preparation of a psychotropic drug.

**Claims for the following Contracting State: ES**

**1.** A process for preparing a compound of the formula (I):

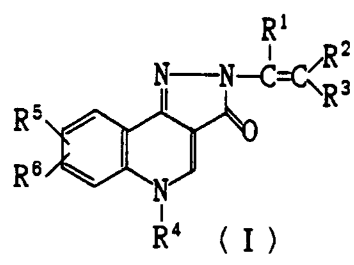

( I )

wherein $R^1$, $R^2$, and $R^3$ each is hydrogen, lower alkyl, cycloalkyl, or phenyl or $R^1$ and $R^2$ taken together may form polymethylene, which may be intervened by one or more of oxygen, sulfur, and/or nitrogen and may have 1 or 2 lower alkyls as substituent: $R^4$ is hydrogen or lower alkyl;
$R^5$ and $R^6$ each is hydrogen, lower alkyl, lower alkyloxy, or halogen or taken together may form methylenedioxy,
which process comprises reacting a compound of the formula (III):

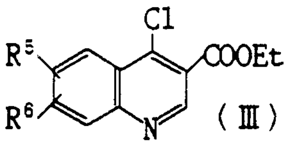
(III)

wherein $R^5$ and $R^6$ each has the same meaning as defined above, with a compound of the formula (IV):

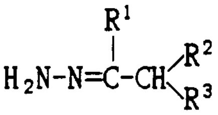
(IV)

wherein $R^1$, $R^2$, and $R^3$ each has the same meaning as defined above, to give the compound of the formula (II):

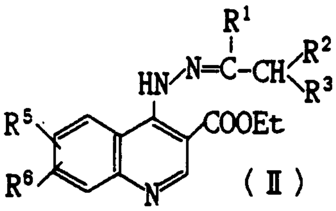
(II)

wherein $R^1$, $R^2$, $R^3$, $R^5$, and $R^6$ each has the same meaning as defined above, which is further subjected to ring closure reaction.

**2.** A process according to claim 1, wherein $R^1$ and $R^2$ taken together form polymethylene.

**3.** A process according to claim 2, wherein polymethylene is intervened by one or more oxygen, sulfur, and/or nitrogen.

**4.** A process according to claim 3, wherein polymethylene is intervened by one or more oxygen.

**5.** A process according to claim 3, wherein polymethylene is intervened by one or more sulfur.

**6.** A process according to claim 3, wherein polymethylene is intervened by one or more nitrogen.

**7.** A process according to claim 3, wherein polymethylene may have 1 or 2 lower alkyls as substituents.

**8.** A method for the preparation of a pharmaceutical composition, wherein a compound prepared according to any of claims 1 to 7 is combined with a pharmaceutically acceptable carrier.

**9.** A method according to claim 8, wherein the pharmaceuitcal composition prepared is useful as a psychotropic drug.

European Patent Office

# EUROPEAN SEARCH REPORT

Application Number

EP 91 11 4680

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 182 165 (SHIONOGI)<br>* claims 1,7 *<br>----- | 1,8 | C07D471/04<br>C07D491/14<br>A61K31/435<br>//(C07D471/04, 231:00,221:00)<br>C07D491/14, 317:00,231:00, 221:00) |
| | | | **TECHNICAL FIELDS SEARCHED (Int. Cl.5)** |
| | | | C07D<br>A61K |

The present search report has been drawn up for all claims

| Place of search | Date of completion of the search | Examiner |
|---|---|---|
| THE HAGUE | 17 JANUARY 1992 | ALFARO FAUS I. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)